# EUROPEAN PATENT SPECIFICATION

(11) **EP 2 162 461 B1**
(45) Date of publication and mention of the grant of the patent: **29.03.2017**
(21) Application number: 08760409.6
(22) Date of filing: 03.06.2008
(51) Int. Cl.: C07K 1/32, C12N 15/62, A61K 38/00

(54) **TARGETED INDUCTION OF AGGREGATION OF PROTEINS WITH CROSS BETA STRUCTURES**
GEZIELTE INDUKTION VON AGGREGATION VON PROTEINEN MIT CROSS-BETA-STRUKTUREN
INDUCTION CIBLÉE DE L'AGRÉGATION DE PROTÉINES AVEC DES STRUCTURES BÊTA CROISÉES

(30) Priority: 04.06.2007 US 933227 P
(43) Date of publication of application: 17.03.2010
(73) Proprietor: VIB VZW, 9052 Gent (BE); Vrije Universiteit Brussel, 1050 Brussel (BE)
(72) Inventor: SCHYMKOWITZ, Joost, B-3391 Meensel-Kiezegem (BE); ROUSSEAU, Frederic, B-1702 Groot-Bijgaarden (BE)
(86) International application number: PCT/EP2008/056825
(87) International publication number: WO 2008/148751

(56) References cited:
- WO-A-2005/118633
- WO-A-2007/008069
- WO-A-2007/008071
- WO-A1-2007/071789
- LÓPEZ DE LA PAZ MANUELA ET AL: "De novo designed peptide-based amyloid fibrils." PROCEEDINGS OF THE NATIONAL ACADEMY OF SCIENCES OF THE UNITED STATES OF AMERICA 10 DEC 2002, vol. 99, no. 25, 10 December 2002 (2002-12-10), pages 16052-16057, XP002495711 ISSN: 0027-8424 cited in the application

## Description

### Field of the invention

The present invention belongs to the field of protein aggregation. The invention discloses a method for interfering with the function of a target protein and uses a non-naturally, user-designed molecule, designated as aggregator, that has a specificity for a target protein and which induces aggregation upon contact with said target protein. The present invention also discloses such aggregator molecules and their use in therapeutic and diagnostic applications.

### Background of the invention

Protein aggregation has become a key topic in both biotechnological and medical sciences. It constitutes the main bottleneck in protein production, narrowing the spectrum of relevant polypeptides obtained by recombinant techniques; it reduces the shelf life and can increase the immunogenicity of polypeptidic drugs; and it is associated with an increasing number of critical human diseases including Alzheimer's disease, spongiform encephalopaties, type II diabetes mellitus and Parkinson's disease. In the last decade data have begun to accumulate suggesting that the composition and the primary structure of a polypeptide determine to a large extent its propensity to aggregate and that small changes may have a huge impact on solubility. The ability to predict the aggregation propensity of a protein from its sequence is of much value as witnessed by the plethora of algorithms able to predict beta-aggregation sequences in proteins. These algorithms are useful in the control of unwanted protein deposition events through specific sequence targeted therapeutics or in the discovery of more soluble variants of proteins of biotechnological interest. It is commonly assumed that not all regions of a polypeptide are equally important in determining its aggregation tendency. In this context, it has been recently proved that very short specific amino acid stretches can act as facilitators or inhibitors of amyloid fibril formation. In the field of functional proteomics there is a need to develop innovative technologies in order to accelerate discoveries and to maximize the potential offered by complementary methods in functional genomics. It would be desirable to possess a flexible technology that can directly target the biological function of a particular extracellular or intracellular protein instead of targeting the mRNA that translates it or manipulating the gene that encodes it. In the present invention we sought to develop a technology to target specific proteins based on aggregation. Thus far, protein aggregation has mainly been studied as an unwanted, disease-causing phenomenon and it is widely accepted that cross-beta mediated aggregation is the most frequently occurring and biologically relevant mechanism of aggregation². Cross-beta aggregation is the term used to indicate that aggregation is nucleated via the formation of intermolecular beta-sheets to which each molecule in the aggregate contributes an identical strand of typically comprising at least three contiguous amino acids. Patent application WO03102187 (Scegen, Pty Ltd) discloses a method for enhancing the activity of a molecule by fusing said molecule with a membrane translocating sequence, whereby the resulting chimeric molecule self-assembles into a higher molecular weight aggregate. US20050026165 (Areté Associates) discloses the use of conformational peptides, able to interact with the beta-sheet conformation of insoluble proteins such as prions, as a diagnostic tool for prion diseases. Patent application WO2007010110 describes the induction of protein aggregation through the use of polycyclic compounds.

### Summary of the Invention

The present invention relates to a technology for the controlled and inducible protein aggregation of specific target proteins. The invention also provides *de novo* designed molecules, herein designated as aggregator molecules, which comprise at least one beta-aggregation region coupled to a binding region with an affinity for a target protein. In a preferred embodiment the aggregator molecule comprises at least one beta-aggregation region that is fused to a region able to bind (or interact with) to the target protein. Upon contact between a chosen target protein and a specifically designed aggregator molecule, a specific co-aggregation occurs between the target and the aggregator resulting in a functional knock-out or a down-regulation of the biological function for said target protein. This protein knock-down is conditional upon the presence of aggregates, which are induced by the presence of the aggregator molecule. An additional advantage is that the strength of the protein interference can be experimentally controlled by varying the number of beta-aggregation regions in the aggregator molecule. The invention does not only provide an efficient research instrument to down-regulate the biological function of a specific extra- or intracellular protein but has also important therapeutic, agricultural and diagnostic applications.

### Figure legends

Figure 1:
The figure shows the residual percentage HRP activity in the supernatant after removal of the co-aggregates by centrifugation (see example 1). The presence of the aggregator (biotinylated peptide) clearly removes the enzyme from the soluble fraction.

### Aims and detailed description of the invention

The present invention is defined in the appended claims. Subject matters which are not encompassed by the scope of the claims do not form part of the presently claimed invention.

In the present invention we have developed a process for down-regulating the biological function of a protein through the use of non-naturally occurring molecules that comprise a region capable of binding to said protein and at least one beta-aggregation sequence. Upon contact with a target protein a co-aggregation occurs between said non-naturally occurring molecule and the target protein. The aggregation withdraws the target from its soluble environment and results in a functional knock-down of the soluble target protein. With 'aggregation' it is understood that aggregation can mean either amorphic or fibrillar (amyloid or cross-beta fibrillar are equivalent terms) aggregation. In fact the kind of beta-aggregation region determines the induction of amorphic or fibrillar aggregation of a target protein.

Thus in one embodiment the invention provides a method for down-regulating the biological function of a protein comprising contacting said protein with a non-naturally occurring molecule comprising a region capable of binding to said protein and at least one beta-aggregation sequence. The wording 'capable of binding to a target protein' means 'capable of interacting with a target protein' or in still other words 'has an affinity for the target protein'. It is understood that said affinity is in the range of micromolar affinity to picomolar affinity, preferably in the range from nanomolar to picomolar affinity. Thus the affinity of an aggregator (or a binding domain thereof capable of binding to a target protein) for a target protein is at least 10⁴ mol⁻¹ (e.g. at least 10⁵, 10⁶, 10⁷, 10⁸, 10⁹, 10¹⁰, 10¹¹ or 10¹² mol⁻¹). It is understood that such a non-naturally occurring molecule is a chimeric molecule which comprises a fusion of two elements: a binding region and at least one beta-aggregation sequence, said two elements have no affinity for each other and/or said elements do not bind each other. No affinity means that the affinity is lower than 10⁴ mol⁻¹. In a preferred embodiment said elements are different from the target protein which one aims to down-regulate (or in an equivalent wording: which one aims to aggregate). In another preferred embodiment said binding region can modulate the function of a target protein. In yet another preferred embodiment said binding region does not modulate the function of a target protein, meaning that the binding region (when binding alone) merely binds the target protein without interfering with the biological function of the target protein. In a particular embodiment a target protein is a fusion protein such as a histidine-fusion, a streptavidin-fusion, or a GFP-fusion.

The term 'binding region' or 'binding domain' typically refers to a molecule that interacts with the target protein. In certain cases a binding domain is a chemical compound (e.g. a small compound with an affinity for at least one target protein) and in certain other cases a binding domain is a polypeptide, in certain other cases a binding domain is a protein domain. A protein binding domain is an element of overall protein structure that is self-stabilizing and often folds independently of the rest of the protein chain. Binding domains vary in length from between about 25 amino acids up to 500 amino acids and more. Many binding domains can be classified into folds and are recognizable, identifiable, 3-D structures. Some folds are so common in many different proteins that they are given special names. Disclosed herein are Rossman folds, TIM barrels, armadillo repeats, leucine zippers, cadherin domains, death effector domains, immunoglobulin-like domains, phosphotyrosine-binding domain, pleckstrin homology domain, src homology 2 domain, the BRCT domain of BRCA1, G-protein binding domains, the Eps 15 homology (EH) domain and the protein-binding domain of p53. Antibodies are the natural prototype of specifically binding proteins with specificity mediated through hypervariable loop regions, so called complementary determining regions (CDR). Although in general, antibody-like scaffolds have proven to work well as specific binders, it has become apparent that it is not compulsory to stick strictly to the paradigm of a rigid scaffold that displays CDR-like loops. In addition to antibodies, many other natural proteins mediate specific high-affinity interactions between domains. Alternatives to immunoglobulins have provided attractive starting points for the design of novel binding (recognition) molecules. The term scaffold refers to a protein framework that can carry altered amino acids or sequence insertions that confer binding to specific target proteins. Engineering scaffolds and designing libraries are mutually interdependent processes. In order to obtain specific binders, a combinatorial library of the scaffold has to be generated. This is usually done at the DNA level by randomizing the codons at appropriate amino acid positions, by using either degenerate codons or trinucleotides. A wide range of different non-immunoglobulin scaffolds with widely diverse origins and characteristics are currently used for combinatorial library display. Some of them are comparable in size to a scFv of an antibody (about 30kDa), while the majority of them are much smaller. Modular scaffolds based on repeat proteins vary in size depending on the number of repetitive units. A non-limiting list of examples comprise binders based on the human 10^{th} fibronectin type III domain, binders based on lipocalins, binders based on SH3 domains, binders based on members of the knottin family, binders based on CTLA-4, T-cell receptors, neocarzinostatin, carbohydrate binding module 4-2, tendamistat, kunitz domain inhibitors, PDZ domains, Src homology domain (SH2), scorpion toxins, insect defensin A, plant homeodomain finger proteins, bacterial enzyme TEM-1 beta-lactamase, Ig-binding domain of Staphylococcus aureus protein A, E. coli colicin E7 immunity protein, E. coli cytochrome b562, ankyrin repeat domains. Also disclosed herein as binding domains are compounds with a specificity for a given target protein, cyclic and linear peptide binders, peptide aptamers, multivalent avimer proteins or small modular immunopharmaceutical drugs, ligands with a specificity for a receptor or a co-receptor, protein binding partners identified in a two-hybrid analysis, binding domains based on the specificity of the biotin-avidin high affinity interaction, binding domains based on the specificity of cyclophilin-FK506 binding proteins. Also disclosed herein are lectins with an affinity for a specific carbohydrate structure.

In yet another embodiment the invention provides a method for down-regulating the biological function of a protein comprising contacting said protein with a non-naturally occurring molecule (or with a chimeric molecule) which comprises part A and part B wherein i) part A is binding region capable of binding to said protein and ii) part B which comprises at least 1 beta-aggregation region. In a particular embodiment said at least one beta-aggregation region consists of at least 3 contiguous amino acids. In a particular embodiment said beta-aggregation region is derived from the target protein. In another particular embodiment said beta-aggregation region is different from the target protein (i.e. it is derived from a different protein). In yet another embodiment said beta-aggregation region is an artificial polypeptide sequence (id est not derived from an existing protein sequence, id est not found in nature). In another particular embodiment a linker is present between parts A and B. A particular type of beta-aggregation region is an amyloid region.

In another embodiment part B of the non-naturally occurring molecule (or chimeric molecule) comprises at least 2 beta-aggregation regions.

The term 'non-naturally occurring molecule' or 'chimeric molecule' or 'chimeric fusion molecule' refers to the fact that such a molecule is man made. For instance, when a molecule is a polypeptide (id est both part A and B are peptides) such polypeptide part B is designed in silico or isolated from a protein in nature (id est a beta-aggregation region) and by fusing (or coupling) said part B to a part A which is a region capable of binding to a protein wherein it is understood that A and B have no affinity for each other (id est do not bind each other or differently worded: do not interact with each other). In still other words the beta-aggregation region (or sequence) fused to a binding region is different from a naturally occurring fusion between part A and B by at least one natural amino acid. Typically, such chimeric molecule will not exist as a contiguous polypeptide in a protein encoded by a gene in a non-recombinant genome.

It should be clear that aggregator molecules can be designed in a modular fashion, by introducing repetition and changing the order of the parts A and B. A list of the following combinations is: an aggregator with the A-B - structure, an aggregator with the B-A - structure, an aggregator with the A-B-A - structure, an aggregator with the B-A-B - structure, an aggregator with the A'-B-A" structure and an aggregator with the B'-A-B" structure wherein a linker (spacer) is optionally present between parts A, A', A" and B, B', B". A, A' and A" are different of similar moieties (e.g. different peptide sequences or different chemical moieties). B, B' and B" are different or similar self association sequences (e.g. B is a beta-aggregation sequence derived from the target protein and B' is a synthetic beta-aggregation sequence). The wording 'down-regulating the function of a protein' means that the normal biological activity of a protein is reduced (inhibited, down-regulated, reduced and disrupted are equivalent words here) or that the protein is withdrawn from its normal biological environment (e.g. a protein which is a normal resident of the endoplasmic reticulum is not present through down-regulation of its function) through induced aggregation (id est a co-aggregation between the aggregator and the target protein). Thus, by applying the method of the invention the function of a protein is disrupted through an aggregation of said protein by contacting said protein with the non-natural molecule of the present invention. Said non-natural molecule is herein designated as 'the aggregator' or the 'aggregator molecule'. Aggregation refers to the fact that a protein which is normally soluble is changed into an insoluble protein or an aggregated protein in its normal biological environment through direct contact or binding with the aggregator. The wording 'down-regulating the function of a protein' can also be interchanged by the wording 'knocking down the function of a protein' or 'negatively interfering with the function of a protein'. The down-regulation of the function of a protein can also mean that a protein is not present anymore in a soluble form in the cell or that a protein is not present anymore in a soluble form in its normal biological environment (e.g. (sub)-cellular or extracellular localization). In addition, it can also mean that the aggregated protein is degraded through the natural clearance mechanisms of the cell and is no longer detectable in soluble or insoluble form. In addition, it can also mean that a transmembrane receptor protein cannot bind its normal ligand anymore through aggregator induced aggregation of said transmembrane protein (e.g. by formation of a fusion between a ligand that normally binds with said growth factor receptor with at least one beta-aggregation domain). Thus the down-regulation of the function of a protein can also mean that a protein which is a normal resident of e.g. the mitochondria is not present there anymore through the method of induced protein aggregation. In a particular embodiment the 'down-regulation of the function of a protein' or 'the negative interference with the function of a protein' or 'knocking down the function of a protein' is at least a 20%, at least a 30%, at least a 40%, at least a 50%, at least a 60%, at least a 70%, at least a 80%, at least a 90%, at least a 95% or even a 100% loss of function as compared to the normal (100%) function of the protein.

The function of a protein or the lack of presence of a protein in its normal biological environment (localization) can conveniently be determined by methods known in the art. For example, depending on the target protein of interest, the function can be determined by measuring the reduced enzymatic activity. The reduced presence of a protein in its normal biological localization can for example be measured by the lack of formation of a complex, the lack of the occurrence of a target protein in a sub-cellular compartment, the presence of the target protein in soluble form, the presence of the target protein in an aggregated (insoluble is an equivalent term here) form. Alternatively, the effect of the down-regulation of a target protein can be measured in a cellular assay (e.g. loss or gain of growth, loss or gain of invasion, loss or gain of proteolytic activity).

In a particular embodiment such normal biological activity (or normal function or normal localization) of a protein can be interfered with intracellularly or extracellularly. 'Intracellularly' refers to the localization of a protein inside the cell of an organism or host (e.g. the cytoplasm, the mitochondria, the lysosome, the vacuole, the nucleus, the chloroplast, the endoplasmic reticulum (ER), the cellular membrane, the mitochondrial membrane, the chloroplast membrane,...). 'Extracellularly' not only refers to the localization of a protein in the extracellular medium of the cell but also refers to proteins which contact the extracellular medium such as a membrane-anchored proteins, a transmembrane protein etc. Examples of extracellular proteins are secreted proteins (e.g. proteases, antibodies and cytokines present in the blood or plasma) or proteins present in the extracellular matrix (e.g. matrix metalloproteins and transmembrane proteins (e.g. a growth factor receptor)).

Cells or hosts which can be targeted with the method of the invention comprise prokaryotic and eukaryotic cells. Examples are viruses, bacteria, yeasts, fungi, protozoa, plants and mammals including humans.

It should be clear that the method of down-regulation the biological function of a protein can be used to interfere with the biological function with 1, 2, 3, 4, 5 or even more proteins simultaneously. Particularly since part A comprises at least one binding region, part A can for example comprise different binding regions each specific for a different protein.

The aggregator used for interference with the biological function of at least one target protein is not naturally present in nature and can be made through chemical synthesis or through recombinant protein expression or through a combination of the latter.

Thus an aggregator molecule comprises at least one beta-aggregation region (thus part B comprises at least one beta-aggregation region). A 'beta-aggregation region' is herein defined as a contiguous sequence of amino acids that has a high tendency to form a tight molecular assembly with identical or very closely related sequences. A 'beta-aggregation region' can also be designated as a 'self-association region'. The wording 'has a high tendency to form a tight molecular assembly' can also be construed as 'has a high affinity'. Affinity is usually translated into values of dissociation (Kd- values). Kd-values between beta-aggregation regions are typically lying between micromolar and nanomolar ranges, but can be sub-nanomolar or supra-micromolar. Examples of beta-aggregation regions are intermolecular beta sheet regions, alpha-helical elements, amyloid regions, hairpin loops, transmembrane sequences and signal sequences. In a particular embodiment at least one beta-aggregation region is present in part B. In another particular embodiment at least two beta-aggregation regions are present in part B. In another particular embodiment 3, 4, 5, 6 or more beta-aggregation regions are present in part B. Said beta-aggregation regions can be interconnected by a linker region (e.g. a spacer of about 2 to about 4 amino acids). The target protein is defined herein as the protein with which one wants to interfere with its function. Thus, in order to make the aggregator specific for at least one protein at least one binding region in part A should be capable of binding to said target protein. It is preferred that the length of a beta-aggregation region consists of at least 3 contiguous amino acids. In a preferred embodiment said region consists of about 3 to about 30 amino acids. In another preferred embodiment said region consists of about 3 to about 25 amino acids. In a particularly preferred embodiment said region consists of about 5 to about 20 amino acids.

Beta-aggregation regions present in part B of the aggregator molecule are optionally coupled with a spacer (or linker) between said beta-aggregation regions. For example, beta-aggregation regions that can be used can be derived from beta-aggregation regions of proteins which do not normally occur in the host (thus some beta-aggregation regions in part B can be taken from an unrelated organism). The nature of the beta-aggregation regions determine the level of inhibition (id est the strength of inhibition) of a target protein through induced aggregation. More than one beta-aggregation region can be used in an aggregator molecule and also synthetic beta-aggregation regions can be used. In a particular embodiment such beta-aggregation regions consist of a synthetic sequence which is not derived from existing proteins and hence does not occur in nature. Examples of such synthetic self association regions are described in López de la Paz M. et al (2002) PNAS 99, 25, p. 16053, table 1.

In the aggregator molecules part B and part A may be optionally linked (or coupled) by means of a linker region (a spacer is an equivalent word). Said linker region can for instance be an unnatural linker made by chemical synthesis (e.g. a flexible linker such as a hydroxy-substituted alkane chain, dextran, polyethylene glycol or the linker can also consist of amino acid homologues) or said linker can exist of natural amino acids such as a poly(threonine) or poly(serine). Preferentially when the linker comprises amino acids, the length of said linker region is between about 3 and about 15 amino acids, more preferably between about 5 and about 10 amino acids. Often a flexible linker can be chosen but it is envisaged that a stiff linker will also work. Flexible linker sequences can be taken from nature, mostly such regions connect domains in naturally occurring proteins, such as the linker between the SH2 and SH3 domains src tyrosine kinase or the linker between the BRCT domains of BRCA1.

The term 'contacting' refers to the process in which the aggregator and the target protein interact. In one form the aggregator is added (e.g. aggregator is present at a particular concentration in a solution) to a sample comprising the target protein. In another form the aggregator molecule is injected into an organism comprising the target protein. Contacting can for example also be carried out through the process of transformation of a cell comprising the target protein, e.g. an isolated cell, e.g. in cell culture, a unicellular microorganism or a cell or a plurality of cells within a multicellular organism. Transformation implies that the aggregator molecule is introduced in a host (e.g. a cell) through commonly known transfection or transformation methods (e.g. by gene transfer techniques including calcium phosphate, DEAE-dextran, electroporation, microinjection, viral methods, the use of cationic liposomes (see for example Feigner, P. L. et al. (1987), Proc. Natl. Acad. Sci USA 84, 7413), commercially available cationic lipid formulations e.g. Tfx 50 (Promega) or Lipofectamin2000 (Life Technologies), particle bombardment, etc.). The aggregator molecule may be encoded by a recombinant vector (e.g. a plasmid, cosmid, viral vector) and can be synthesized inside a host. In an alternative embodiment the aggregator molecule can be introduced into a cell through carrier-mediated delivery, e.g. by liposomal carriers or nano-particles or by injection. In yet another alternative embodiment the aggregator molecule can enter a cell through a sequence which mediates cell penetration (or cell translocation). In the latter case the aggregator molecule is further modified through the recombinant or synthetic attachment of a cell penetration sequence. Thus, the aggregator molecule (e.g. as a polypeptide) may be further fused or chemically coupled to a sequence facilitating transduction of the fusion or chemical coupled proteins into prokaryotic or eukaryotic cells. Sequences facilitating protein transduction are known to the person skilled in the art and include Protein Transduction Domains. Preferably, said sequence is selected from the group comprising of the HIV TAT protein, a polyarginine sequence, penetratin and pep-1. Still other commonly used cell-permeable peptides (both natural and artificial peptides) are disclosed in Joliot A. and Prochiantz A. (2004) Nature Cell Biol. 6 (3) 189-193.

In a particular embodiment the aggregator essentially consists of amino acids. A "Polypeptide" refers to a polymer in which the monomers are amino acids and are joined together through amide bonds, alternatively referred to as a peptide. When the amino acids are alpha-amino acids, either the L-optical isomer or the D-optical isomer can be used. Additionally, unnatural amino acids, for example, beta-alanine, phenylglycine and homoarginine are also included. Commonly encountered amino acids that are not gene-encoded may also be used in the present invention. All or part of the amino acids used in the aggregators may be either the D-or L-isomer. In addition, other peptidomimetics are also useful in the present invention. We specifically refer the review of the development and use of peptidomimetics as antagonists for protein-protein interactions from Sillerud LO and Larson RS (2005) Curr Protein Pept Sci. 6(2):151-69. Furthermore, D-amino acids can be added to the peptide sequence to stabilize turn features (especially in the case of glycine). In another approach alpha, beta, gamma or delta turn mimics (such as alpha, beta, gamma, or delta dipeptides can be employed to mimic structural motifs and turn features in a peptide and simultaneously provide stability from proteolysis and enhance other properties such as, for example, conformational stability and solubility.

### Isolation of a beta-aggregation region from a target protein:

Beta-aggregation sequences are often hydrophobic but this is not always the case. For example, the beta-aggregation (or self-associating) regions of the yeast prions are rather polar. In fact cross-beta aggregation of an amino acid region derived from a polypeptide or protein can be initiated when (1) it has a high hydrophobicity, (2) it has a good β-sheet propensity, (3) it has a low net charge and (4) it is solvent-exposed. Thus, beta-aggregation protein regions ('segment' is an equivalent term for 'region') are most often buried in the folded state and are not exposed to the solvent. The latter is confirmed by the experimental finding that in many globular proteins, aggregation occurs during refolding or under conditions in which denatured or partially folded states are significantly populated, i.e. at high concentration or as a result of destabilizing conditions or mutations.

Based on these findings computer algorithms were developed that are able to predict beta-aggregation regions ("β-aggregating stretches or segments" is an equivalent wording) in proteins. One such algorithm, TANGO, is based on a statistical mechanics algorithm that considers the three physico-chemical parameters described above but also considers competition between different structural conformations: beta-turn, alpha-helix, beta-sheet aggregates and the folded state (Fernandez-Escamilla, AM et al (2004) Nat. Biotechnol. 22, 1302-1306, especially the Methods section on pages 1305 and 1306 and also the Supplementary Notes 1 and 2 of the same article for further details on the methods and the data sets used for the calibration and the testing of the TANGO algorithm). Thus, beta-aggregation regions present in target proteins are obtainable by computer algorithms such as TANGO. Another algorithm that determines beta-aggregation regions, in particular amyloid regions is SALSA which is described by Zibaee S et al (2007) Protein Sc. 16(5):906-18. Yet another algorithm that can determine amyloid structure regions in proteins is PASTA which is described by Trovato A et al (2006) PLoS Comput Biol 2(12): e170. Yet another algorithm is Aggrescan (Conchillo-Sole O et al (2007) BMC Bioinformatics 8:65. The latter algorithm also predicts aggregation-prone segments (id est beta-aggregation regions) in proteins.

Beta-aggregation regions are often buried inside the core of the target proteins¹⁰, effectively shielding the peptide from intermolecular association by an energy barrier corresponding to the stability of the target proteins¹¹. In its normal environment (e.g. cytoplasm, extracellular matrix) the target protein has assistance from molecular chaperones that assist the protein in keeping its functional, monomeric form¹². TANGO can be accessed on the World Wide Web at http://tango.embl.de/. The zyggregator algorithm is another example (Pawar AP et al (2005) J. Mol. Biol. 350, 379-392). These algorithms identify aggregation prone sequences by comparing the aggregation propensity score of a given amino acid sequence with an average propensity calculated from a set of sequences of similar length.

The present invention uses these beta-aggregation regions for the preparation of aggregator molecules which are used for the specific induction of protein aggregation. The B-part of the aggregator molecules comprises at least 1 aggregation region. It is possible to control the strength of the protein interference (the strength of protein interference is for example the % of loss of biological function of a target protein when said protein or cell comprising said protein is contacted with a specific aggregator molecule) through the incorporation of more than one aggregation region in the B-part of the aggregator molecule. Indeed, aggregation regions with a low TANGO score (typically between 5 % to about 20%) can be repeated in the B-part of the aggregator to 2, 3, 4 or more aggregation regions. As an alternative embodiment 1, 2 or 3 or 4 or more different aggregation regions with a low TANGO score can be incorporated into the B-part of the aggregator. As another alternative embodiment 1, 2, 3, 4 or more synthetic aggregation regions can be combined into the B-part to enhance the down-regulation of a target protein.

Thus in another embodiment the invention provides a non-naturally occurring molecule capable of aggregating a target protein. In a particular embodiment said non-naturally molecule is proteinaceous in nature. Proteinaceous means that the molecule comprises L-amino acids or D-amino acids or a mixture of L- and D- amino acids or a combination of natural amino acids and peptidomimetics.

In yet another embodiment the invention provides a non-naturally occurring molecule comprising at least one beta-aggregation region isolated from a protein domain capable of being soluble in water wherein said beta-aggregation region is fused to a moiety that prevents aggregation of said beta-aggregation region.

In yet another embodiment the invention provides a non-naturally occurring molecule comprising at least one beta-aggregation region fused to a region capable of binding to a target protein.

In a specific embodiment the aggregator molecules that comprise at least one beta-aggregation region fused to a binding region capable of interacting with a target protein are polypeptides.

In another specific embodiment the invention provides a recombinant vector comprising a polynucleotide encoding such aggregator molecules.

In another specific embodiment the aggregator molecules of the invention are used as a medicament.

### Therapeutic applications of the aggregator molecules

Proteins are responsible for biological activities ranging from numerous enzymatic reactions, over transduction of signals to providing structure. Changes in protein structure, abundance or activity are at the root cause or many diseases. Many drugs act via specific interference with one or a limited number of proteins. The present invention provides a method to develop a novel class of compounds able to specifically interfere with a target protein of choice by using a binding region capable of interacting with a target protein. These novel compounds are designated as aggregators.

Thus, in yet another embodiment the invention provides the use as a medicament of a non-naturally occurring molecule comprising at least one beta-aggregation region wherein said beta-aggregation region is fused to a region capable if interacting with a target protein. Said binding region prevents auto-aggregation of said aggregator molecule.

Said aggregator molecules can be used for treating diseases and/or in the manufacturing of a medicament to treat diseases, such as cancer, associated with the aberrant expression of at least one target protein, such as an oncogenic protein. The term 'aberrant expression' refers to for example the (over)expression of an oncogenic protein in the case of cancer, it also includes the expression of a dominant negative protein, the undesired localization of a particular protein or splice variant of a particular protein, the undesired expression of a particular splice variant of a particular protein, the higher activity of a mutant protein or the higher activity of a particular protein.

In a particular embodiment the "aberrant expression" refers to the unwanted presence of a post-translationally modified protein or to the undesired presence of a non-post-translationally modified protein. Post-translational modifications alter the physico-chemical properties of the modified amino acids, and as such they have the potential of altering the aggregation tendency of a given polypeptide segment that can be exploited to specifically target the form that has the strongest aggregation tendency. So if a post-translational modification significantly decreases the aggregation tendency of the beta-aggregation region, then interference will be most efficient with the unmodified protein. In contrast, in case of post-translational modifications that increase the aggregation tendency of the beta-aggregation region, then interference will be most efficient with the modified protein. Based on the hydrophobicity alone, it is assumed that modifications such as phosphorylation and glycosylation will decrease aggregation tendency, whereas lipid attachment will increase aggregation tendency.

The target protein to which the aggregator molecule of the invention is directed may be associated with a pathological condition. For example, the protein may be a pathogen-associated protein e.g. a viral protein, a tumor-associated protein, or an autoimmune disease-associated protein. In one aspect, the invention features an aggregator molecule for use in a method of treating a subject at risk for or afflicted with unwanted cell proliferation, e.g. malignant or non-malignant cell proliferation. The method includes: providing an aggregator molecule, e.g. an aggregator having a structure as described herein, wherein said aggregator molecule is capable of interfering with (inhibiting) the function and/or presence of a protein that promoted unwanted cell proliferation and administering said aggregator to a subject, preferably a human subject, thereby treating the subject.

In a preferred embodiment, the protein is a growth factor or growth factor receptor, a kinase (e.g. a protein tyrosine, serine or threonine kinase), an adaptor protein, a protein from the G protein coupled receptor super-family, or a transcription factor. In a preferred embodiment, the aggregator molecule interferes with the biological function of the PDGF-beta protein, and thus can be used to treat a subject having or at risk for a disorder characterized by unwanted PDGF-beta expression, e.g. testicular and lung cancers. In another preferred embodiment the aggregator inhibits (knocks down) the function and/or presence of the Erb-B protein, and thus can be used to treat a subject having or at risk for a disorder characterized by unwanted Erb-B expression, e. g. breast cancer. In another preferred embodiment, the aggregator inhibits the function (or "interferes with the function" which is equivalent) of (or interferes with the presence of) the Src protein, and thus can be used to treat a subject having or at risk for a disorder characterized by unwanted Src expression, e.g. colon cancers. In another preferred embodiment, the aggregator inhibits the function and/or presence of the CRK protein, and thus can be used to treat a subject having or at risk for a disorder characterized by unwanted CRK expression, e.g. colon and lung cancers. In another preferred embodiment, the aggregator interferes with the function and/or presence of the GRB2 protein, and thus can be used to treat a subject having or at risk for a disorder characterized by unwanted GRB2 expression, e.g. squamous cell carcinoma. In another preferred embodiment the aggregator molecule interferes with the function and/or presence of the RAS gene, and thus can be used to treat a subject having or at risk for a disorder characterized by unwanted RAS expression e.g. pancreatic, colon and lung cancers, and chronic leukemia. In another preferred embodiment the aggregator molecule interferes with the function and/or presence of the MEKK protein, and thus can be used to treat a subject having or at risk for a disorder characterized by unwanted MEKK expression, e.g. squamous cell carcinoma, melanoma or leukemia. In another preferred embodiment the aggregator molecule interferes with the function and/or presence of the JNK protein, and thus can be used to treat a subject having or at risk for a disorder characterized by unwanted JNK expression, e.g. pancreatic or breast cancers. In another preferred embodiment, the aggregator molecule interferes with the function and/or presence of the RAF protein and thus can be used to treat a subject having or at risk for a disorder characterized by unwanted RAP expression, e.g. lung cancer or leukemia. In another preferred embodiment, the aggregator molecule interferes with the function and/or presence of the Erkl/2 protein, and thus can be used to treat a subject having or at risk for a disorder characterized by unwanted Erkl/2 expression, e.g. lung cancer. In another preferred embodiment the aggregator molecule interferes with the function and/or presence of the PCNA (p2I) protein, and thus can be used to treat a subject having or at risk for a disorder characterized by unwanted PCNA expression, e.g. lung cancer. In another preferred embodiment, the aggregator molecule interferes with the function and/or presence of the MYB protein, and thus can be used to treat a subject having or at risk for a disorder characterized by unwanted MYB expression, e.g., colon cancer or chronic myelogenous leukemia. In a preferred embodiment the aggregator molecule interferes with the function and/or presence of the c-MYC protein, and thus can be used to treat a subject having or at risk for a disorder characterized by unwanted c-MYC expression, e.g., Burkitt's lymphoma or neuroblastoma. In another preferred embodiment the aggregator molecule interferes with the function and/or presence of the JUN protein, and thus can be used to treat a subject having or at risk for a disorder characterized by unwanted JUN expression, e. g. ovarian, prostate or breast cancers. In another preferred embodiment the aggregator molecule interferes with the function and/or presence of the FOS protein, and thus can be used to treat a subject having or at risk for a disorder characterized, by unwanted FOS expression, e.g. skin or prostate cancers. In another preferred embodiment, the aggregator molecule inhibits the function and/or presence of the BCL-2 protein, and thus can be used to treat a subject having or at risk for a disorder characterized by unwanted BCL-2 expression, e.g., lung or prostate cancers or non-Hodgkin lymphoma. In another preferred embodiment, the aggregator molecule interferes with the function and/or presence of the Cyclin D protein, and thus can be used to treat a subject having or at risk for a disorder characterized by unwanted Cyclin D expression, e.g. esophageal and colon cancers. In another preferred embodiment, the aggregator molecule interferes with the function and/or presence of the VEGF protein, and thus can be used to treat a subject having or at risk for a disorder characterized by unwanted VEGF expression, e.g., esophageal, colon cancers or pathological angiogenesis. In a preferred embodiment, the aggregator molecule interferes with the function and/or presence of the EGFR protein, and thus can be used to treat a subject having or at risk for a disorder characterized by unwanted EGFR expression, e.g. breast cancer. In another preferred embodiment the aggregator molecule interferes with the function and/or presence of the Cyclin A protein, and thus can be used to treat a subject having or at risk for a disorder characterized by unwanted Cyclin A expression, e.g., lung and cervical cancers. In another preferred embodiment the aggregator molecule interferes with the function and/or presence of the Cyclin E protein, and thus can be used to treat a subject having or at risk for a disorder characterized by unwanted Cyclin E expression, e.g. lung and breast cancers. In another preferred embodiment the aggregator molecule interferes with the function and/or presence of the WNT-1 protein, and thus can be used to treat a subject having or at risk for a disorder characterized by unwanted WNT-1 expression, e.g., basal cell carcinoma. In another preferred embodiment the aggregator molecule interferes with the function and/or presence of the beta-catenin protein, and thus can be used to treat a subject having or at risk for a disorder characterized by unwanted beta-catenin expression, e.g., adenocarcinoma or hepatocellular carcinoma. In another preferred embodiment the aggregator molecule interferes with the function and/or presence of the c-MET protein, and thus can be used to treat a subject having or at risk for a disorder characterized by unwanted c-MET expression, e.g. hepatocellular carcinoma. In another preferred embodiment the aggregator molecule interferes with the function and/or presence of the protein kinase C (PKC) protein, and thus can be used to treat a subject having or at risk for a disorder characterized by unwanted PKC expression, e.g., breast cancer. In another preferred embodiment, the aggregator molecule interferes with the function and/or presence of the NFKappa-B protein, and thus can be used to treat a subject having or at risk for a disorder characterized by unwanted NFKappa-B expression, e.g., breast cancer. In another preferred embodiment, the aggregator molecule interferes with the function and/or presence of the STAT3 protein, and thus can be used to treat a subject having or at risk for a disorder characterized by unwanted STAT3 expression, e.g. prostate cancer. In another preferred embodiment the aggregator molecule interferes with the function and/or presence of the survivin protein, and thus can be used to treat a subject having or at risk for a disorder characterized by unwanted survivin expression, e.g. cervical or pancreatic cancers. In another preferred embodiment the aggregator molecule interferes with the function and/or presence of the Her2/Neu protein, and thus can be used to treat a subject having or at risk for a disorder characterized by unwanted Her2/Neu expression, e.g. breast cancer. In another preferred embodiment the aggregator molecule interferes with the function and/or presence of the topoisomerase I protein, and thus can be used to treat a subject having or at risk for a disorder characterized by unwanted topoisomerase I expression, e.g. ovarian and colon cancers. In another preferred embodiment, the aggregator molecule interferes with the function and/or presence of the topoisomerase II alpha protein, and thus can be used to treat a subject having or at risk for a disorder characterized by unwanted topoisomerase II expression, e.g., breast and colon cancers.

In another aspect, the invention provides an aggregator molecule for use in a method of treating a subject, e.g. a human, at risk for or afflicted with a disease or disorder that may benefit by angiogenesis inhibition, e.g. cancer. The method includes: providing an aggregator molecule e.g., an aggregator molecule having a structure described herein, which aggregator molecule can inhibit (or interfere with the function) a protein which mediates angiogenesis and administering the aggregator molecule to a subject, thereby treating the subject. In a preferred embodiment, the aggregator molecule interferes with the function and/or presence of the alpha v-integrin protein, and thus can be used to treat a subject having or at risk for a disorder characterized by unwanted alpha v-integrin, e.g. brain tumors or tumors of epithelial origin. In another preferred embodiment, the aggregator molecule interferes with the function and/or presence of the Flt-1 receptor protein, and thus can be used to treat a subject having or at risk for a disorder characterized by unwanted Flt-I receptors e.g. cancer and rheumatoid arthritis. In another preferred embodiment, the aggregator molecule interferes with the function and/or presence of the tubulin protein, and thus can be used to treat a subject having or at risk for a disorder characterized by unwanted tubulin, e. g., cancer and retinal neovascularization.

In another aspect, the invention provides an aggregator molecule for use in a method of treating a subject infected with a virus or at risk for or afflicted with a disorder or disease associated with a viral infection. The method includes: providing an aggregator molecule, e.g. an aggregator molecule having a structure described herein, which aggregator molecule is homologous to and can silence, a viral protein or a cellular protein which mediates viral function, e.g. entry or growth; and administering said aggregator molecule to a subject, preferably a human subject, thereby treating the subject. As such the invention provides methods of using aggregators for the manufacture of a medicament to treat patients infected by viruses including the Human Papilloma Virus, Human Immunodeficiency Virus (HIV), Hepatitis B Virus (HBV), Hepatitis A Virus (HAV), Hepatitis C Virus (HCV), Respiratory Syncytial Virus (RSV), Herpes Simplex Virus (HSV), Cytomegalovirus (CMV), Epstein Barr-Virus (EBV), a rhinovirus, West Nile Virus, Tick-borne encephalitis virus, measles virus (MV), or poliovirus.

In another aspect, the invention features aggregator molecules for use in methods of treating a subject infected with a pathogen, e.g. a bacterial, amoebic, parasitic, or fungal pathogen. The method includes: providing an aggregator molecule, e.g. an aggregator molecule having a structure described herein, wherein said aggregator molecule is capable of interfering with the function of a pathogenic protein derived from said pathogen and administering said aggregator molecule to a subject, preferably a human subject, thereby treating the subject. The target protein from the pathogen can be one involved in growth, cell wall synthesis, protein synthesis, transcription, energy metabolism (e.g. the Krebs cycle) or toxin production. Thus, the present invention provides for an aggregator molecule for use in a method of treating patients infected by for example Plasmodium falciparum, Mycobacterium ulcerans, Mycobacterium tuberculosis, Mycobacterium leprae, Staphylococcus aureus, Streptococcus pneumoniae, Streptococcus pyogenes, Chlamydia pneumoniae, or Mycoplasma pneumoniae.

In another aspect, the invention provides an aggregator molecule for use in a method of treating a subject, e.g. a human, at risk for or afflicted with a disease or disorder characterized by an unwanted immune response, e.g. an inflammatory disease or disorder, or an autoimmune disease or disorder. The method includes: providing an aggregator molecule, e.g. an aggregator molecule having a structure described herein, which aggregator molecule is capable of inhibiting (down-regulating) the function and/or presence of a protein which mediates an unwanted immune response, and administering said aggregator molecule to a subject, thereby treating the subject. In a preferred embodiment, the disease or disorder is an ischemia or reperfusion injury, e.g. ischemia reperfusion or injury associated with acute myocardial infarction, unstable angina, cardiopulmonary bypass, surgical intervention (e.g. angioplasty, such as percutaneous transluminal coronary angioplasty), a response to a transplanted organ or tissue (e.g. transplanted cardiac or vascular tissue), or thrombolysis. In another preferred embodiment, the disease or disorder is restenosis, e.g. restenosis associated with surgical intervention (e.g., angioplasty, such as percutaneous transluminal coronary angioplasty). In another preferred embodiment, the disease or disorder is Inflammatory Bowel Disease e.g. Crohn's Disease or Ulcerative Colitis. In another preferred embodiment, the disease or disorder is inflammation associated with an infection or injury. In another preferred embodiment, the disease or disorder is asthma, lupus, multiple sclerosis, diabetes, e.g. type II diabetes, arthritis, e.g. rheumatoid or psoriatic. In another preferred embodiment the aggregator molecule interferes with the function of an integrin or co-ligand thereof, e.g. VLA4, VCAM, ICAM. In another preferred embodiment the aggregator molecule interferes with the function of a selectin or co-ligand thereof, e.g. P-selectin, E-selectin (ELAM), L-selectin, or P-selectin glycoprotein-(PSGL1). In another preferred embodiment the aggregator molecule interferes with the function of a component of the complement system, e.g., C3, C5, C3aR, C5aR, C3 convertase, C5 convertase. In another preferred embodiment, the aggregator molecule interferes with the function of a chemokine or receptor thereof e.g. TNF-α, IL-1α, IL-1, IL-2, IL-2R, IL-4, IL-4R, IL-5, IL-6, IL-8, TNFRI, TNFRII, IgE, SCYA11 or CCR3.

In another aspect, the invention provides an aggregator molecule for use in a method of treating a subject, e.g., a human, at risk for or afflicted with acute pain or chronic pain. The method includes providing an aggregator molecule, e.g. an aggregator molecule having a structure described herein, which aggregator molecule is capable of interfering with a protein which mediates the processing of pain and administering said aggregator molecule to a subject, thereby treating the subject. In another preferred embodiment the aggregator molecule interferes with the function of a component of an ion channel. In another particularly preferred embodiment, the aggregator molecule interferes with the function of a neurotransmitter receptor or ligand.

In another aspect, the invention features an aggregator molecule for use in a method of treating a subject e.g. a human, at risk for or afflicted with a neurological disease or disorder. The method includes providing an aggregator molecule, e.g. an aggregator molecule having a structure described herein, which aggregator molecule is capable of interfering with a protein which mediates a neurological disease or disorder and administering said aggregator molecule to a subject, thereby treating the subject. In particular embodiments said diseases (or disorders) which can be treated include Alzheimer's Disease (in this case the aggregator molecule interferes with the function of a secretase which leads to the processing of APP e.g. a protein involved in the gamma-secretase complex (e.g. presenilin protein 1 or 2, an Aph1 protein, nicastrin, BACE1 or BACE2). The aggregator inhibits the processing of APP and prevents the formation of insoluble amyloid beta. The same strategy can be used to prevent and/or to treat other neurodegenerative diseases such as Huntington's disease, a spinocerebellar ataxia (e.g. SCA1, SCA2, SCA3 (Machado-Joseph disease), SCA7 or SCA8).

Thus in one aspect the invention provides a method for the production or manufacture of a medicament or a pharmaceutical composition comprising at least one aggregator molecule and furthermore mixing said aggregator molecule with a pharmaceutically acceptable carrier. In a preferred embodiment the aggregator molecule is a polypeptide and can be made synthetically or as a recombinant protein. The recombinant protein may be manufactured using recombinant expression systems comprising bacterial cells, yeast cells, animal cells, insect cells, plant cells or transgenic animals or plants. The recombinant protein may be purified by any conventional protein purification procedure close to homogeneity and/or be mixed with additives.

The administration of a pharmaceutical composition comprising an aggregator molecule may be by way of oral, inhaled, transdermal or parenteral (including intravenous, intratumoral, intraperitoneal, intramuscular, intracavity, and subcutaneous) administration. The active compound may be administered alone or preferably formulated as a pharmaceutical composition. A unit dose will normally contain 0.01 to 500 mg, for example 0.01 to 50 mg, or 0.01 to 10 mg, or 0.05 to 2 mg of compound or a pharmaceutically acceptable salt thereof. Unit doses will normally be administered once or more than once a day, for example 2, 3, or 4 times a day, more usually 1 to 3 times a day, such that the total daily dose is normally in the range of 0.0001 to 10 mg/kg; thus a suitable total daily dose for a 70 kg adult is 0.01 to 700 mg, for example 0.01 to 100 mg, or 0.01 to 10 mg or more usually 0.05 to 10 mg.

It is preferred that the compound or a pharmaceutically acceptable salt thereof is administered in the form of a unit-dose composition, such as a unit dose oral, parenteral, transdermal or inhaled composition. Such compositions are prepared by admixture and are suitably adapted for oral, inhaled, transdermal or parenteral administration, and as such may be in the form of tablets, capsules, oral liquid preparations, powders, granules, lozenges, reconstitutable powders, injectable and infusable solutions or suspensions or suppositories or aerosols.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well-known methods in the art. Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate. These solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents. Oral formulations also include conventional sustained release formulations, such as tablets or granules having an enteric coating.

Preferably, compositions for inhalation are presented for administration to the respiratory tract as a snuff or an aerosol or solution for a nebulizer, or as a microfine powder for insufflation, alone or in combination with an inert carrier such as lactose. In such a case the particles of active compound suitably have diameters of less than 50 microns, preferably less than 10 microns, for example between 1 and 5 microns, such as between 2 and 5 microns. Alternatively, coated nanoparticles can be used, with a particle size between 30 and 500 nm. A favored inhaled dose will be in the range of 0.05 to 2 mg, for example 0.05 to 0.5 mg, 0.1 to 1 mg or 0.5 to 2 mg.

For parenteral administration, fluid unit dose forms are prepared containing a compound of the present invention and a sterile vehicle. The active compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum. Parenteral suspensions are prepared in substantially the same manner except that the compound is suspended in the vehicle instead of being dissolved and sterilised by exposure to ethylene oxide before suspending in the sterile vehicle. Advantageously, a surfactant or wetting agent is included in the composition to facilitate uniform distribution of the active compound. Where appropriate, small amounts of bronchodilators for example sympathomimetic amines such as isoprenaline, isoetharine, salbutamol, phenylephrine and ephedrine; xanthine derivatives such as theophylline and aminophylline and corticosteroids such as prednisolone and adrenal stimulants such as ACTH may be included.

As is common practice, the compositions will usually be accompanied by written or printed directions for use in the medical treatment concerned.

In a preferred embodiment the aggregator molecule further comprises a protein transduction domain. It has been shown that a series of small protein domains, termed protein transduction domains (PTDs), cross biological membranes efficiently and independently of transporters or specific receptors, and promote the delivery of peptides and proteins into cells. For example, the TAT protein from human immunodeficiency virus (HIV-1) is able to deliver biologically active proteins in vivo. Similarly, the third alpha-helix of Antennapedia homeodomain, and VP22 protein from herpes simplex virus promote the delivery of covalently linked peptides or proteins into cells (reviewed in Ford KG et al (2001) Gene Ther. 8, 1-4). Protein delivery based on a short amphipathic peptide carrier, Pep-1, is efficient for delivery of a variety of peptides and proteins into several cell lines in a fully biologically active form, without the need for prior chemical covalent coupling (Morris MC et al, (2001) Nat. Biotechnol. 19, 1173-1176). The capacity of VP22 chimeric proteins to spread from the primary transduced cell to surrounding cells can improve gene therapy approaches (Zender L et al (2002) Cancer Gene Ther. 9, 489-496). Sequences, facilitating protein transduction are known to the person skilled in the art and include, but are not limited to Protein Transduction Domains. Preferably, said sequence is selected from the group comprising of the HIV TAT protein, a polyarginine sequence, penetratin and pep-1. Still other commonly used cell-permeable peptides (both natural and artificial peptides) are disclosed in Joliot A. and Prochiantz A. (2004) Nature Cell Biol. 6 (3) 189-193.

A second aspect of a pharmaceutical composition is the use of a nucleotide sequence encoding the aggregator molecules. In case a nucleic acid sequence encoding the aggregator molecule is used, said medicament is preferably intended for delivery of said nucleic acid into the cell, in a gene therapy treatment. A large number of delivery methods are well known to those of skill in the art. Preferably, the nucleic acids are administered for *in vivo* or *ex vivo* gene therapy uses. Non-viral vector delivery systems include DNA plasmids, naked nucleic acid, and nucleic acid complexed with a delivery vehicle such as a liposome. Viral vector delivery systems include DNA and RNA viruses, which have either episomal or integrated genomes after delivery to the cell. Methods of non-viral delivery of nucleic acids include lipofection, microinjection, biolistics, virosomes, liposomes, immunoliposomes, polycation or lipid:nucleic acid conjugates, naked DNA, artificial virions, and agent-enhanced uptake of DNA. Lipofection is described in, e.g., US Pat. No. 5,049,386, US Pat No. 4,946,787; and US Pat. No. 4,897,355 and lipofection reagents are sold commercially (e.g., Transfectam^{™} and Lipofectin^{™}). Cationic and neutral lipids that are suitable for efficient receptor-recognition lipofection of polynucleotides include those of Flegner, WO 91/17424, WO 91/16024. Delivery can be to cells (*ex vivo* administration) or target tissues (*in vivo* administration). The preparation of lipid: nucleic acid complexes, including targeted liposomes such as immunolipid complexes, is well known to one of skill in the art (*see, e.g*., Crystal, 1995; Blaese *et al.,* 1995; Behr, 1994; Remy *et al.,* 1994; Gao and Huang, 1995; U.S. Pat. Nos. 4,186,183, 4,217,344, 4,235,871, 4,261,975, 4,485,054, 4,501,728, 4,774,085, 4,837,028, and 4,946,787).

The use of RNA or DNA viral based systems for the delivery of nucleic acids take advantage of highly evolved processes for targeting a virus to specific cells in the body and trafficking the viral payload to the nucleus. Viral vectors can be administered directly to patients (*in vivo*) or they can be used to treat cells *in vitro* and the modified cells are administered to patients (*ex vivo*). Conventional viral based systems for the delivery of nucleic acids include amongst others retroviral, lentivirus, adenoviral, adeno-associated and herpes simplex virus vectors for gene transfer. Viral vectors are currently the most efficient and versatile method of gene transfer in target cells and tissues. Integration in the host genome is possible with the retrovirus, lentivirus, and adeno-associated virus gene transfer methods, often resulting in long-term expression of the inserted transgene. Additionally, high transduction efficiencies have been observed in many different cell types and target tissues. In cases where transient expression of the nucleic acid is preferred, adenoviral based systems, including replication deficient adenoviral vectors may be used. Adenoviral based vectors are capable of very high transduction efficiency in many cell types and do not require cell division. With such vectors, high titer and levels of expression have been obtained. This vector can be produced in large quantities in a relatively simple system. Adeno-associated virus ("AAV") vectors, including recombinant adeno-associated virus vectors are also used to transduce cells with target nucleic acids, e.g., in the *in vitro* production of nucleic acids and peptides, and for *in vivo* and *ex vivo* gene therapy procedures (*see, e.g.,* U.S. Patent No. 4,797,368; WO 93/24641; Kotin, 1994; The construction of recombinant AAV vectors is described in a number of publications, including U.S. Pat. No. 5,173,414; Hermonat & Muzyczka, 1984; Samulski *et al*., 1989).

Gene therapy vectors can be delivered *in vivo* by administration to an individual patient, typically by systemic administration (e.g., intravenous, intraperitoneal, intramuscular, intratracheal, subdermal, or intracranial infusion) or topical application. In a particular embodiment the invention also envisages the use of a hydrodynamic gene therapeutic method. Hydrodynamic gene therapy is disclosed in US6627616 (Mirus Corporation, Madison) and involves the intravascular delivery of non-viral nucleic acids encoding an aggregator whereby the permeability of vessels is increased through for example the application of an increased pressure inside said vessel or through the co-administration of vessel permeability increasing compounds such as for example papaverine.

Alternatively, vectors can be delivered to cells *ex vivo*, such as cells explanted from an individual patient (e.g., lymphocytes, bone marrow aspirates, tissue biopsy) or universal donor hematopoietic stem cells, followed by reimplantation of the cells into a patient, usually after selection for cells which have incorporated the vector. *Ex vivo* cell transfection for diagnostics, research, or for gene therapy (e.g., via re-infusion of the transfected cells into the host organism) is well known to those of skill in the art. In a preferred embodiment, cells are isolated from the subject organism, transfected with a nucleic acid (gene or cDNA), and re-infused back into the subject organism (e.g., patient). Various cell types suitable for *ex vivo* transfection are well known to those of skill in the art (*see, e.g.,* Freshney *et al.,* 1994 and the references cited therein for a discussion of how to isolate and culture cells from patients).

In yet another embodiment the method of protein aggregation (or protein interference) of the invention may be used for determining the function of a protein in a cell or an organism being capable of mediating protein interference. The cell can be a prokaryotic cell or can be a eukaryotic cell or can be a cell line, e.g. a plant cell or an animal cell, such as a mammalian cell, e.g. an embryonic cell, a pluripotent stem cell, a tumor cell, e.g. a teratocarcinoma cell or a virus-infected cell. The organism is preferably a eukaryotic organism, e.g. a plant or an animal, such as a mammal, particularly a human.

The target protein to which the aggregator molecule of the invention is directed may be associated with a pathological condition. For example, the protein may be a pathogen-associated protein, e.g. a viral protein, a tumor-associated protein or an autoimmune disease-associated protein. The target protein may also be a heterologous gene expressed in a recombinant cell or a genetically altered organism. By inhibiting the function of such a protein valuable information and therapeutic benefits in the agricultural field or in the medicine or veterinary medicine field may be obtained. In a particularly preferred embodiment the method of the invention is used with an eukaryotic cell or a eukaryotic non-human organism exhibiting a target protein-specific knockout phenotype comprising an at least partially deficient expression of at least one endogenous target protein wherein said cell or organism is contacted with at least one aggregator molecule capable of inhibiting the function of at least one endogenous target protein or with a vector encoding at least aggregator molecule capable of interfering with the function and/or presence of at least one endogenous protein. It should be noted that the present invention also allows a target-specific knockout of several different endogenous proteins due to the specificity of the aggregator molecule.

Protein-specific knockout phenotypes of cells or non-human organisms, particularly of human cells or non-human mammals may be used in analytic procedures, e.g. in the functional and/or phenotypical analysis of complex physiological processes such as analysis of proteomes. For example, one may prepare the knock-out phenotypes of human proteins in cultured cells which are assumed to be regulators of alternative splicing processes. Among these proteins are particularly the members of the SR splicing factor family, e.g. ASF/SF2, SC35, SRp20, SRp40 or SRp55. Further, the effect of SR proteins on the mRNA profiles of predetermined alternatively spliced genes such as CD44 may be analysed.

Using the protein based knockout technologies described herein, the expression of an endogenous target protein may be inhibited in a target cell or a target organism. The endogenous protein may be complemented by an exogenous target nucleic acid coding for the target protein or a variant or mutated form of the target protein, e.g. a gene or a cDNA, which may optionally be fused to a further nucleic acid sequence encoding a detectable peptide or polypeptide, e.g. an affinity tag, particularly a multiple affinity tag. Variants or mutated forms of the target protein differ from the endogenous target protein in that they differ from the endogenous protein by amino acid substitutions, insertions and/or deletions of single or multiple amino acids. The variants or mutated forms may have the same biological activity as the endogenous target protein. On the other hand, the variant or mutated target protein may also have a biological activity, which differs from the biological activity of the endogenous target protein, e.g. a partially deleted activity, a completely deleted activity, an enhanced activity etc. The complementation may be accomplished by co-expressing the polypeptide encoded by the exogenous nucleic acid, e.g. a fusion protein comprising the target protein and the affinity tag and the aggregator molecule for knocking out the endogenous protein in the target cell. This co-expression may be accomplished by using a suitable expression vector expressing both the polypeptide encoded by the exogenous nucleic acid, e.g. the tag-modified target protein and the aggregator molecule or alternatively by using a combination of expression vectors or alternatively the aggregator molecule may contact the target cell from the outside of the cell. Proteins and protein complexes which are synthesized *de novo* in the target cell will contain the exogenous protein, e.g. the modified fusion protein. In order to avoid suppression of the exogenous protein function with the aggregator molecule, the exogenous protein must have sufficient amino acid differences in the aggregation region that is selected for the design of the aggregator molecule. Alternatively, the endogenous target protein may be complemented by corresponding proteins from other species, or the endogenous target protein may be complemented by a splice form of said target protein. The combination of knockout of an endogenous protein and rescue by using mutated, e.g. partially deleted exogenous target has advantages compared to the use of a knockout cell. Further, this method is particularly suitable for identifying functional domains of the target protein.

In a further preferred embodiment a comparison, e.g. of gene expression profiles and/or proteomes and/or phenotypic characteristics of at least two cells or organisms is carried out. These organisms are selected from: (i) a control cell or control organism without target protein inhibition, (ii) a cell or organism with target protein inhibition and (iii) a cell or organism with target protein inhibition plus target protein complementation by an exogenous target nucleic acid encoding said target protein.

The methods of the invention are also suitable in a procedure for identifying and/or characterizing pharmacological agents, e.g. identifying new pharmacological agents from a collection of test substances and/or characterizing mechanisms of action and/or side effects of known pharmacological agents. Thus, the present invention also relates to a system for identifying and/or characterizing pharmacological agents acting on at least one target protein comprising: (a) a eukaryotic cell or a eukaryotic non-human organism capable of expressing at least one endogenous target gene coding for said target protein, (b) at least one aggregator molecule capable of inhibiting the expression of said at least one endogenous target gene, and (c) a test substance or a collection of test substances wherein pharmacological properties of said test substance or said collection are to be identified and/or characterized. Further, the system as described above preferably comprises: (d) at least one exogenous target nucleic acid coding for the target protein or a variant or mutated form or splice form of the target protein wherein said exogenous target protein differs from the endogenous target protein on the amino acid level of the aggregation regions such that the function of the exogenous target protein is substantially less inhibited by the aggregator molecule than the expression of the endogenous protein.

In addition, the invention also comprises cells and organisms comprising an aggregator molecule. An organism can for example be a transgenic plant which carries the genetic information that encodes an aggregator. Such a transgenic plant is in a preferred embodiment a silenced plant (id est in which a particular target protein is down-regulated because of the presence of a specific aggregator in a sub-set of cells or organs or present in all cells and organs of said plant). Cells comprising an aggregator can be produced by contacting said cells or by electroporation of said cells with a particular aggregator molecule. In a particular embodiment cells comprising an aggregator are generated through transfection (or transformation) wherein the aggregator is encoded by a recombinant expression vector such as a plasmid or a viral vector.

### ISOLATION: SEPARATION and DETECTION

In another embodiment the invention provides a method to isolate a protein from a sample comprising contacting said sample with a non-naturally occurring molecule comprising at least one beta-aggregation region fused to a region capable of binding to a target protein and isolating the resulting co-aggregated molecule-protein complex from said sample.

In other words the invention provides a method for the isolation of a protein from a sample comprising:
- contacting said protein with a non-naturally occurring molecule which comprises part A and part B wherein i) part A is a region capable of binding to a protein and ii) part B which comprises at least 1 beta-aggregation region consisting of at least 3 contiguous amino acids and wherein a linker is optionally present between parts A and B and
- isolating the resulting co-aggregated molecule-protein complex from said sample.

### SEPARATION

In a further embodiment the method for the isolation of at least one protein further comprises the separation of at least one protein from a sample.

One application of the separation of at least one protein from a sample is the removal (or depletion) of highly abundant proteins from a sample. Indeed, a major challenge in protein target discovery and validation is how to specifically dissect complex protein samples (e.g. plasma, urine, cerebrospinal fluid) and measure trace targets. The abundant proteins are often 6-10 orders of magnitude more concentrated than low abundant proteins. Thus, highly abundant proteins must be removed to detect and measure trace proteins of medical importance. Since albumin, IgG, antitrypsin, IgA, transferrin and haptoglobin make up approximately 90% of the total protein content in human serum, there is a critical need for diagnostic tools to rapidly deplete these unwanted abundant proteins and unmask the less abundant, low molecular weight protein biomarkers. Several methods are already used in the art: 1) immunoglobulin G (IgG) as affinity reagents to capture and separate abundant protein targets, 2) immunoglobulin yolk (IgY) are IgG-like antibodies isolated from egg yolks of immunized birds, 3) pre-fractionation is used to separate a mixture of proteins into different fractions to remove certain proteins in the original mixture, and 4) protein A and protein G are bacterial cell wall proteins with a specificity to IgG antibodies, hence protein A and G affinity resins provide a removal of IgG and 5) IgG- and IgY-microbeads are used for protein detection.

### DETECTION

In another specific embodiment the method for the isolation of at least one protein further comprises the detection of at least one protein in said molecule-protein complex.

Detection can be carried out by separating the aggregator molecule-target protein complex by for example electrophoresis, column chromatography, centrifugation, filtration, electrostatic attraction, magnetic or paramagnetic attraction, mass spectrometry and the like.

In a particular embodiment the aggregator molecule consists of a small chemical compound or a drug (e.g. an active medicinal compound for which the protein targets are unknown) fused to a beta-aggregation region. Such aggregator molecule can be used to identify (or to detect) drug targets in a complex protein mixture (e.g. a cell lysate).

The most broadly used biodetection technologies are based on the use of antibodies. Antibodies recognize and bind to other molecules based on their shape and physicochemical properties. Antibodies are highly suited for detecting small quantities of target proteins in the presence of complex mixtures of proteins. The present invention shows that the use of aggregator molecules (with a specificity and affinity (recognition) for at least one specific protein) - when the binding region is different from an antibody binding element - can be used as an alternative for the use of antibodies (as the recognition element) for the specific capture of target proteins. Indeed, aggregator molecules can be used in numerous applications in which antibodies typically are used. To name only a few, applications are envisaged in diagnosis, micro-analytics, forensics and in the specific detection of pathogens.

For the detection and separation applications of the invention the aggregator molecule, with a specificity for a given target protein, can be bound to a carrier. A carrier can be a flat surface such as plastic or nitrocellulose or a chromatographic column but is preferably a bead such as microsphere beads. Binding (coupling) of the aggregator can be mediated via part A (the binding region with an affinity for a particular protein) or via part B (the at least one beta-aggregation region). A general discussion on various types of beads and microspheres, which serve the purpose of binding the aggregator molecules, is described on pages 9 and 10 of US6682940.

In a particular embodiment the aggregator molecule is bound on a carbohydrate type of carrier, e.g. cellulose or agarose. The aggregator can be covalently coupled to said carbohydrate carrier with a cross-linking agent such as glutaraldehyde.

In another particular embodiment the aggregator is bound on a support such as cellulose, glass or a synthetic polymer. Covalent attachment can be carried out via amino acid residues of part A or B and an azide, carbodiimide, isocyanate or other chemical derivatives.

In yet another particular embodiment the support is a porous silanised glass micro bead. The aggregator can be covalently bonded via its peptide amine groups (by Schiff reaction followed by reduction with sodium borohydride) to aldehyde groups formed by periodate oxidation of glycidoxypropylsilane groups chemically linked to the silica atoms (this coupling is described in Sportsman and Wilson (1980) Anal. Chem. 52, 2013-2018).

In a specific embodiment the carrier part is enveloped by a proteinaceous film to which the aggregator is crosslinked (see claims 1-50 and examples relating to the carrier in US4478946). In another specific embodiment the support is a fluorescent bead such as a fluorescent latex particle. The patent US4550017, and especially page 4 therein, describes fluorescent compounds which can be used for the manufacturing of fluorescent beads.

In another specific embodiment the beads vary in size and may also contain or be impregnated with fluorescent dyes. Because of varying sizes and dyes of the beads multiple proteins can be detected and quantitated in a single reaction. Procedures for the development of such beads are described in US6159748.

In yet another particular embodiment the coupling between the bead and the aggregator is via a poly(threonine), a poly(serine), dextran or poly(ethylene glycol). Examples 6, 7, 8 and 9 of US6399317 illustrate how this coupling can be carried out.

In yet another particular embodiment the support is a magnetic bead. Magnetic beads, coupling between the magnetic beads and a protein agent (such as a polypeptidic aggregator) and their uses are described on page 8 of application US6489092.

### Definitions

Unless defined otherwise, all technical and scientific terms used herein have the same meaning as commonly understood by those of ordinary skill in the art to which the invention belongs. Although any methods and materials similar or equivalent to those described herein can be used in the practice or testing of the present invention, preferred methods and materials are described. For the purposes of the present invention, the following terms are defined below.

The articles "a" and "an" are used herein to refer to one or to more than one (i.e. to at least one) of the grammatical object of the article. By way of example, "a target protein" means one target protein or more than one target protein.

As used herein, the term "about" refers to a quantity, level, value, dimension, size, or amount that varies by as much as 30%, preferably by as much as 20%, and more preferably by as much as 10% to a reference quantity, level, value, dimension, size, or amount.

"Bifunctional crosslinking reagent" means a reagent containing two reactive groups, the reagent thereby having the ability to covalently link two elements such as part A and part B of the aggregator molecule. The reactive groups in a crosslinking reagent typically belong to the classes of functional groups including succinimidyl esters, maleimides and haloacetamides such as iodoacetamides. Throughout this specification, unless the context requires otherwise, the words "comprise", "comprises" and "comprising" will be understood to imply the inclusion of a stated step or element or group of steps or elements but not the exclusion of any other step or element or group of steps or elements.

By "expression vector" or "recombinant vector" is meant any autonomous genetic element capable of directing the synthesis of an aggregator molecule encoded by the vector. Such expression vectors are known to practitioners in the art.

By "derivative" is meant an aggregator molecule that has been derived from the basic sequence by modification, for example by conjugation or complexing with other chemical moieties (e.g. pegylation) or by post-translational modification techniques as would be understood in the art. The term "derivative" also includes within its scope alterations that have been made to a parent sequence including additions, or deletions that provide for functionally equivalent molecules.

By "effective amount", in the context of modulating an activity or of treating or preventing a condition is meant the administration of that amount of an aggregator molecule to an individual in need of such modulation, treatment or prophylaxis, either in a single dose or as part of a series, that is effective for modulation of that effect or for treatment or prophylaxis of that condition. The effective amount will vary depending upon the health and physical condition of the individual to be treated, the taxonomic group of individual to be treated, the formulation of the composition, the assessment of the medical situation, and other relevant factors. It is expected that the amount will fall in a relatively broad range that can be determined through routine trials.

By "isolated" is meant material that is substantially or essentially free from components that normally accompany it in its native state. For example, an "isolated polypeptide", as used herein, refers to a polypeptide, which has been purified from the sequences which flank it in a naturally-occurring state, e.g., a beta-aggregation sequence which has been removed from the sequences that are normally adjacent to said sequence. A beta-aggregation sequence can be generated by amino acid chemical synthesis or can be generated by recombinant production.

The term "oligonucleotide" as used herein refers to a polymer composed of a multiplicity of nucleotide units (deoxyribonucleotides or ribonucleotides, or related structural variants or synthetic analogues thereof) linked via phosphodiester bonds (or related structural variants or synthetic analogues thereof). An oligonucleotide is typically rather short in length, generally from about 10 to 30 nucleotides, but the term can refer to molecules of any length, although the term "polynucleotide" or "nucleic acid" is typically used for large oligonucleotides. The term "polynucleotide" or "nucleic acid" as used herein designates mRNA, RNA, cRNA, cDNA or DNA. The term typically refers to oligonucleotides greater than 30 nucleotides in length.

The term "recombinant polynucleotide" as used herein refers to a polynucleotide formed in vitro by the manipulation of nucleic acid into a form not normally found in nature. For example, the recombinant polynucleotide may be in the form of an expression vector. Generally, such expression vectors include transcriptional and translational regulatory nucleic acid operably linked to the nucleotide sequence.

By "operably linked" is meant that transcriptional and translational regulatory nucleic acids are positioned relative to a polypeptide-encoding polynucleotide in such a manner that the polynucleotide is transcribed and the polypeptide is translated.

The terms "subject" or "individual" or "patient", used interchangeably herein, refer to any subject, particularly a vertebrate subject, and even more particularly a mammalian subject, for whom therapy or prophylaxis is desired. Suitable vertebrate animals that fall within the scope of the invention include, but are not restricted to, primates, avians, fish, reptiles, livestock animals (e.g., sheep, cows, horses, donkeys, pigs), laboratory test animals (e.g., rabbits, mice, rats, guinea pigs, hamsters), companion animals (e.g., cats, dogs) and captive wild animals (e.g., foxes, deer, dingoes). However, it will be understood that the aforementioned terms do not imply that symptoms are present.

By "pharmaceutically acceptable carrier" is meant a solid or liquid filler, diluent or encapsulating substance that can be safely used in topical or systemic administration to a patient.

"Polypeptide", "peptide" and "protein" are used interchangeably herein to refer to a polymer of amino acid residues and to variants and synthetic analogues of the same. Thus, these terms apply to amino acid polymers in which one or more amino acid residues is a synthetic non-naturally occurring amino acid, such as a chemical analogue of a corresponding naturally occurring amino acid, as well as to naturally-occurring amino acid polymers.

By "recombinant polypeptide" is meant a polypeptide made using recombinant techniques, i.e., through the expression of a recombinant or synthetic polynucleotide. When the chimeric polypeptide or biologically active portion thereof is recombinantly produced, it is also preferably substantially free of culture medium, i.e., culture medium represents less than about 20%, more preferably less than about 10%, and most preferably less than about 5% of the volume of the protein preparation.

The term "sequence identity" as used herein refers to the extent that sequences are identical on a nucleotide-by-nucleotide basis or an amino acid-by-amino acid basis over a window of comparison. Thus, a "percentage of sequence identity" is calculated by comparing two optimally aligned sequences over the window of comparison, determining the number of positions at which the identical nucleic acid base (e.g., A, T, C, G, I) or the identical amino acid residue (e.g., Ala, Pro, Ser, Thr, Gly, Val, Leu, Ile, Phe, Tyr, Trp, Lys, Arg, His, Asp, Glu, Asn, Gln, Cys and Met) occurs in both sequences to yield the number of matched positions, dividing the number of matched positions by the total number of positions in the window of comparison (i.e., the window size), and multiplying the result by 100 to yield the percentage of sequence identity. For the purposes of the present invention, "sequence identity" will be understood to mean the "match percentage" calculated by the DNASIS computer program (Version 2.5 for windows; available from Hitachi Software engineering Co., Ltd., South San Francisco, Calif., USA) using standard defaults as used in the reference manual accompanying the software. "Similarity" refers to the percentage number of amino acids that are identical or constitute conservative substitutions. Similarity may be determined using sequence comparison programs such as GAP (Deveraux et al. 1984, Nucleic Acids Research 12, 387-395). In this way, sequences of a similar or substantially different length to those cited herein might be compared by insertion of gaps into the alignment, such gaps being determined, for example, by the comparison algorithm used by GAP.

The term "transformation" means alteration of the genotype of an organism, for example a bacterium, yeast or plant, by the introduction of a foreign or endogenous nucleic acid. Vectors for transformation include plasmids, retroviruses and other animal viruses, YACs (yeast artificial chromosome), BACs (bacterial artificial chromosome) and the like. By "vector" is meant a polynucleotide molecule, preferably a DNA molecule derived, for example, from a plasmid, bacteriophage, yeast or virus, into which a polynucleotide can be inserted or cloned. A vector preferably contains one or more unique restriction sites and can be capable of autonomous replication in a defined host cell including a target cell or tissue or a progenitor cell or tissue thereof, or be integrable with the genome of the defined host such that the cloned sequence is reproducible. Accordingly, the vector can be an autonomously replicating vector, i.e., a vector that exists as an extrachromosomal entity, the replication of which is independent of chromosomal replication, e.g., a linear or closed circular plasmid, an extrachromosomal element, a minichromosome, or an artificial chromosome. The vector can contain any means for assuring self-replication. Alternatively, the vector can be one which, when introduced into the host cell, is integrated into the genome and replicated together with the chromosome(s) into which it has been integrated. A vector system can comprise a single vector or plasmid, two or more vectors or plasmids, which together contain the total DNA to be introduced into the genome of the host cell, or a transposon. The choice of the vector will typically depend on the compatibility of the vector with the host cell into which the vector is to be introduced. In a preferred embodiment, the vector is preferably a viral or viral-derived vector, which is operably functional in animal and preferably mammalian cells. The vector can also include a selection marker such as an antibiotic resistance gene that can be used for selection of suitable transformants. Examples of such resistance genes are known to those of skill in the art and include the nptll gene that confers resistance to the antibiotics kanamycin and G418 (Geneticin®) and the hph gene which confers resistance to the antibiotic hygromycin B. Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, useful methods and materials are described below. Other features and advantages of the invention will be apparent from the detailed description and from the claims.

### Examples

### 1. Depletion of an enzyme from solution

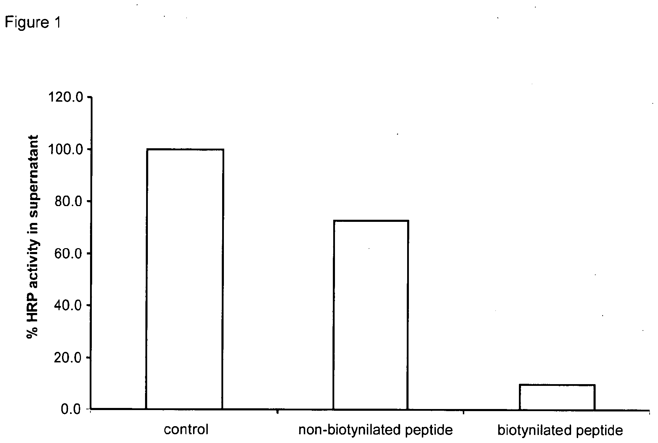

In this example an aggregator was constructed by fusing biotin as a binding region coupled to a beta-aggregation region. This aggretator was used to deplete
an N-terminal fusion protein between Horse Radish Peroxidase (HRP) and streptavidin (a 60 kDa protein streptavidin from the bacterium *Streptomyces avidinii*)*.* HRP is an enzyme that converts 3,3',5,5'-tetramethylbenzidine (TMB) into a blue product that absorbs light efficiently at 370 nm. Biotin (as a binding element) has a strong affinity for streptavidin (the dissociation constant (Kd) of the biotin-streptavidin complex is on the order of ∼10⁻¹⁵ mol/L). The TANGO algorithm was used to identify a beta-aggregating amino acid sequence from beta-galactosidase from E. coli. This beta-aggregation sequence was synthesised (obtained from Jerini Peptide Technologies, Germany) and coupled to a biotin molecule (as an N-terminal fusion) resulting in an aggregator with the following sequence: Biotin-ALAVVLQ-NH2. In order to show depletion between the fusion protein HRP-streptavidin from solution due to co-aggregation with the aggregator, we co-incubated the aggregator with the streptavidin-HRP fusion protein. The aim of this experiment is that via the biotin-streptavidin interaction, the HRP enzyme will co-aggregate with the aggregating peptide, causing a drop in HRP activity in the soluble fraction. To this end, a streptavidin-HRP stock solution (AbD Serotec, product number 710005) was diluted 1:20,000 in PBS and incubated with the aggregator at a final concentration of 1µM. The samples were incubated overnight with agitation at 750rpm at room temperature. A control sample consisting of the fusion protein SA-HRP alone in identical buffer (PBS plus 10% DMSO) was included. In addition, a control sample of the non- biotinylated beta-aggregation sequence was also included to demonstrate specificity. After overnight incubation, co-aggregated material was removed from the sample by centrifugation for 15 min at 17,000 g in a cooled bench top microfuge at 10 °C. Of the supernatant, 10 µL was recovered and added in a 96 well plate (Falcon, 353072) to 90µL of TMB solution. This mixture was incubated at room temperature for 1 minute, after which the colorimetric reaction was arrested by the addition of 100 µL of 2 M H₂SO₄. The absorbance of the resulting yellow product was measured at OD₄₅₀ₙₘ using an Ultra Microplate Reader (BioTEK, ELₓ808IU). Figure 1 shows the residual percentage HRP activity in the supernatant after removal of the aggregates by centrifugation (see above). The presence of the aggregator (biotynilated peptide) clearly removes the enzyme from the soluble fraction.

### 2. Depletion of a monoclonal antibody from solution

We constructed an aggregator molecule wherein part B consists of three synthetic beta-aggregation regions with short linkers of two amino acids (STLIVL-QN-STVIFE-QN-STVIFE) interconnecting said beta-aggregation regions. Said three beta-aggregation regions are hexapeptides which have a strong tendency to aggregate. Note: in the text of this invention all amino acid sequences are depicted starting from the amino-terminal part and read in the direction of the carboxy-terminal part - thus, "STLIVL" reads as "NH2-STLIVL-COOH"). Part B of the synthetic interferor molecule was N-terminally fused to a binding region (part A) that prevents auto-aggregation of said beta-aggregation regions and brings said beta-aggregation regions in direct contact with the environment (here the cytosol of *E. coli*). (Fig. 1 depicts the structure of the synthetic aggregator design). Said binding region is the NusA protein, which is frequently used as a solubilising tag in recombinant protein production¹³, fused N-terminally with a polyhistidine tag. The resulting synthetic interferor molecule (A-B-histidine tag structure) was made and purified in a recombinant way in *E. coli.*

In order to deplete a monoclonal antibody from solution via co-aggregation with the aggregator, we employed a fusion protein consisting of a mouse monoclonal antibody with a specificity for polyhistidine (6xHis) fused to the enzyme horse radish peroxidase (HRP). The HRP enzyme provides a readout system for the presence of the antibody (see example 1). By co-incubating the anti-his mAb with the his-tagged aggregator, we aimed to remove the mAb (anti His-HRP) from solution by co-aggregation with the his-tagged aggregator. For this purpose, anti His-HRP was diluted 1:5000 in PBS and incubated with the aggregator at a final concentration of 1 ng/mL. The samples were incubated overnight in an Eppendorf Thermomixer with agitation (750rpm) at both room temperature and 37°C. Control samples consisting of anti His-HRP alone in identical buffer were also included. After incubation, aggregated material was removed from the sample by centrifugation (15min, 10°C, 17,000g) in a bench top microfuge. 5 x 101µl of each supernatant was quickly recovered into a 96 well plate (Falcon, 353072). 90µl TMB solution was added and after incubation at room temperature for 1 minute the reaction was stopped by the addition of 100uL 2M H₂SO4. The absorbance of the resulting yellow product was measured at OD450nm using an Ultra Microplate Reader (BioTEK, ELₓ808IU). Incubation overnight at RT with the aggregating construct at 10 pg/µL resulted in a drop of HRP activity to 20.5% of the value observed for the control (wherein no aggregator present was present).

### References

1. Dobson, C. M. Protein-misfolding diseases: Getting out of shape. Nature 418, 729-730 (2002).
2. Dobson, C. M. Principles of protein folding, misfolding and aggregation. Semin Cell Dev Biol 15, 3-16 (2004).
3. Nelson, R. et al. Structure of the cross-beta spine of amyloid-like fibrils. Nature 435, 773-8 (2005).
4. Makin, O. S., Atkins, E., Sikorski, P., Johansson, J. & Serpell, L. C. Molecular basis for amyloid fibril formation and stability. Proc Natl Acad Sci U S A 102, 315-20 (2005).
5. Hamada, D., Yanagihara, I. & Tsumoto, K. Engineering amyloidogenicity towards the development of nanofibrillar materials. Trends Biotechnol 22, 93-7 (2004).
6. Fernandez-Escamilla, A. M., Rousseau, F., Schymkowitz, J. & Serrano, L. Prediction of sequence-dependent and mutational effects on the aggregation of peptides and proteins. Nat Biotechnol 22, 1302-6 (2004).
7. Chiti, F., Stefani, M., Taddei, N., Ramponi, G. & Dobson, C. M. Rationalization of the effects of mutations on peptide and protein aggregation rates. Nature 424, 805-8 (2003).
8. Pawar, A. P. et al. Prediction of "aggregation-prone" and "aggregation-susceptible" regions in proteins associated with neurodegenerative diseases. J Mol Biol 350, 379-92 (2005).
9. Lopez de la Paz, M. & Serrano, L. Sequence determinants of amyloid fibril formation. Proc Natl Acad Sci U S A 101, 87-92 (2004).
10. Linding, R., Schymkowitz, J., Rousseau, F., Diella, F. & Serrano, L. A comparative study of the relationship between protein structure and beta-aggregation in globular and intrinsically disordered proteins. J Mol Biol 342, 345-53 (2004).
11. Clark, L. A. Protein aggregation determinants from a simplified model: cooperative folders resist aggregation. Protein Sci 14, 653-62 (2005).
12. Barral, J. M., Broadley, S. A., Schaffar, G. & Hartl, F. U. Roles of molecular chaperones in protein misfolding diseases. Semin Cell Dev Biol 15, 17-29 (2004).
13. De Marco, V., Stier, G., Blandin, S. & de Marco, A. The solubility and stability of recombinant proteins are increased by their fusion to NusA. Biochem Biophys Res Commun 322, 766-71 (2004).
14. Houry, W. A., Frishman, D., Eckerskorn, C., Lottspeich, F. & Hartl, F. U. Identification of in vivo substrates of the chaperonin GroEL. Nature 402, 147-54 (1999).
15. Bairoch, A. et al. The Universal Protein Resource (UniProt). Nucleic Acids Res 33, D154-9 (2005).
16. Kopp, J. & Schwede, T. The SWISS-MODEL Repository of annotated three-dimensional protein structure homology models. Nucleic Acids Res 32, D230-4 (2004).
17. Guex, N. & Peitsch, M. C. SWISS-MODEL and the Swiss-PdbViewer: An environment for comparative protein modeling. Electrophoresis 18, 2714-2723 (1997).

## Claims

1. A chimeric molecule comprising a region capable of binding to a protein and at least one beta-aggregation sequence for use in down-regulating the biological function of the protein wherein a co-aggregation occurs between said chimeric molecule and the protein, wherein said use is *in vivo* and wherein said region capable of binding to a protein is an antibody.

2. An *in vitro* method for down-regulating the biological function of a protein comprising contacting said protein with a chimeric molecule comprising a region capable of binding to said protein and at least one beta-aggregation sequence wherein a co-aggregation occurs between said chimeric molecule and the protein, wherein said region capable of binding to a protein is an antibody or, wherein if the protein to be downregulated is a fusion protein consisting of the protein and a moiety fused thereto, said region capable of binding to a protein and said moiety are a pair selected from an antibody and its corresponding antigen, and biotin and avidin or streptavidin.

3. The molecule for use according to claim 1 or method according to claim 2 wherein said beta-aggregation sequence consists of at least 3 contiguous amino acids.

4. The molecule for use or method according to any one of claims 1 to 3 wherein a linker is present between said beta-aggregation sequence and said binding element.

5. The molecule for use or method according to claim 4 wherein said linker is a polypeptide or is of non-polypeptide nature.

6. The molecule for use or method according to any one of claims 1 to 5 wherein said molecule is a polypeptide and is encoded by a nucleotide sequence present on a recombinant vector and which, upon transformation to a cell or organism, produces said polypeptide in said cell or organism.

7. A method to isolate a protein from a sample comprising contacting said sample with a chimeric molecule comprising a region capable of binding to said protein and at least one beta-aggregation sequence and isolating the resulting co-aggregated molecule-protein complex from said sample, wherein said region capable of binding to said protein is an antibody or, wherein if the protein to be isolated is a fusion protein consisting of the protein and a moiety fused thereto, said region capable of binding to a protein and said moiety are a pair selected from an antibody and its corresponding antigen, and biotin and avidin or streptavidin.

8. A method according to claim 7 further comprising the detection of a protein in said sample.

## Patentansprüche

1. Chimäres Molekül, das eine Region, die an ein Protein binden kann, und mindestens eine beta-Aggregationssequenz umfasst, zur Verwendung beim Herunterregulieren der biologischen Funktion des Proteins, wobei eine Koaggregation zwischen dem chimären Molekül und dem Protein auftritt, wobei die Verwendung *in vivo* ist und wobei die Region, die an ein Protein binden kann, ein Antikörper ist.

2. *In-vitro*-Verfahren zum Herunterregulieren der biologischen Funktion eines Proteins, wobei das Verfahren das Inkontaktbringen des Proteins mit einem chimären Molekül umfasst, das eine Region, die an das Protein binden kann, und mindestens eine beta-Aggregationssequenz umfasst, wobei eine Koaggregation zwischen dem chimären Molekül und dem Protein auftritt, wobei die Region, die an ein Protein binden kann, ein Antikörper ist oder wobei, wenn das Protein, das herunterreguliert werden soll, ein Fusionsprotein ist, das aus dem Protein und einer daran fusionierten Einheit besteht, die Region, die an ein Protein binden kann, und die Einheit ein Paar sind, das aus einem Antikörper und seinem entsprechenden Antigen und Biotin und Avidin oder Streptavidin ausgewählt ist.

3. Molekül zur Verwendung nach Anspruch 1 oder Verfahren nach Anspruch 2, wobei die beta-Aggregationssequenz aus mindestens 3 angrenzenden Aminosäuren besteht.

4. Molekül zur Verwendung oder Verfahren nach einem der Ansprüche 1 bis 3, wobei ein Linker zwischen der beta-Aggregationssequenz und dem Bindeelement vorliegt.

5. Molekül zur Verwendung oder Verfahren nach Anspruch 4, wobei der Linker ein Polypeptid ist oder von einer Nicht-Polypeptid-Beschaffenheit ist.

6. Molekül zur Verwendung oder Verfahren nach einem der Ansprüche 1 bis 5, wobei das Molekül ein Polypeptid ist und von einer Nukleotidsequenz kodiert wird, die an einem rekombinanten Vektor vorliegt und bei Transformation in eine Zelle oder einen Organismus das Polypeptid in der Zelle oder dem Organismus produziert.

7. Verfahren zum Isolieren eines Proteins aus einer Probe, wobei das Verfahren das Inkontaktbringen der Probe mit einem chimären Molekül, das eine Region, die an das Protein binden kann, und mindestens eine beta-Aggregationssequenz umfasst, und das Isolieren des resultierenden koaggregierten Molekül-Protein-Komplex aus der Probe umfasst, wobei die Region, die an das Protein binden kann, ein Antikörper ist oder wobei, wenn das Protein, das isoliert werden soll, ein Fusionsprotein ist, das aus dem Protein und einer daran fusionierten Einheit besteht, die Region, die an ein Protein binden kann, und die Einheit ein Paar sind, das aus einem Antikörper und seinem entsprechenden Antigen und Biotin und Avidin oder Streptavidin ausgewählt ist.

8. Verfahren nach Anspruch 7, das weiterhin den Nachweis eines Proteins in der Probe umfasst.

## Revendications

1. Molécule chimère comprenant une région capable de se fixer à une protéine et au moins une séquence de bêta-agrégation destinée à être utilisée dans la régulation à la baisse de la fonction biologique de la protéine, dans laquelle une co-agrégation se produit entre ladite molécule chimère et la protéine, dans laquelle ladite utilisation est in vivo et dans laquelle ladite région capable de se fixer à une protéine est un anticorps.

2. Procédé in vitro de régulation à la baisse de la fonction biologique d'une protéine comprenant la mise en contact de ladite protéine avec une molécule chimère comprenant une région capable de se fixer à ladite protéine et au moins une séquence de bêta-agrégation, dans lequel une co-agrégation se produit entre ladite molécule chimère et la protéine, dans lequel ladite région capable de se fixer à une protéine est un anticorps, ou dans lequel si la protéine devant être régulée à la baisse est une protéine de fusion constituée de la protéine et d'un fragment fusionné à celle-ci, ladite région capable de se fixer à une protéine et ledit fragment sont une paire sélectionnée parmi un anticorps et son antigène correspondant, et de la biotine et de l'avidine ou de la streptavidine.

3. Molécule destinée à être utilisée selon la revendication 1 ou procédé selon la revendication 2, dans laquelle/lequel ladite séquence de bêta-agrégation est constituée d'au moins 3 acides aminés contigus.

4. Molécule destinée à être utilisée ou procédé selon l'une quelconque des revendications 1 à 3, dans laquelle/lequel un liant est présent entre ladite séquence de bêta-agrégation et ledit élément de fixation.

5. Molécule destinée à être utilisée ou procédé selon la revendication 4, dans laquelle/lequel ledit liant est un polypeptide ou est de nature non polypeptidique.

6. Molécule destinée à être utilisée ou procédé selon l'une quelconque des revendications 1 à 5, dans laquelle/lequel ladite molécule est un polypeptide et est codée par une séquence nucléoditique présente sur un vecteur recombiné et qui, lors de la transformation vers une cellule ou un organisme, produit ledit polypeptide dans ladite cellule ou ledit organisme.

7. Procédé pour isoler une protéine d'un échantillon comprenant mettre en contact ledit échantillon avec une molécule chimère comprenant une région capable de se fixer à ladite protéine et au moins une séquence de bêta-agrégation, et isoler le complexe molécule-protéine co-agrégé résultant dudit échantillon, dans lequel ladite région capable de se fixer à ladite protéine est un anticorps, ou dans lequel si la protéine devant être isolée est une protéine de fusion constituée de la protéine et d'un fragment fusionné à celle-ci, ladite région capable de se fixer à une protéine et ledit fragment sont une paire sélectionnée parmi un anticorps et son antigène correspondant, et de la biotine et de l'avidine ou de la streptavidine.

8. Procédé selon la revendication 7, comprenant en outre la détection d'une protéine dans ledit échantillon.


### SEQUENCE LISTING

<110> VIB VZW
   VRIJE UNIVERSITEIT BRUSSEL
<120> Means and methods for inducing protein aggregation
<130> JSC/RPR/V263
<150> US 60/933,227
   <151> 2007-06-04
<160> 1
<170> PatentIn version 3.5
<210> 1
   <211> 22
   <212> PRT
   <213> Artificial Sequence
<220>
   <223> Part B of aggregator molecule
<400> 1
   Ser Thr Leu Ile Val Leu Gln Asn Ser Thr Val Ile Phe Glu Gln Asn
   1               5                   10                  15
   Ser Thr Val Ile Phe Glu
               20